# EUROPEAN PATENT SPECIFICATION

(11) **EP 1 459 068 B1**
(45) Date of publication and mention of the grant of the patent: **18.08.2010**
(21) Application number: 02806133.1
(22) Date of filing: 21.11.2002
(51) Int. Cl.: G01N 33/558

(54) **INTERNAL CALIBRATION SYSTEM FOR FLOW-THROUGH ASSAYS**
INTERNES KALIBRIERSYSTEM FÜR DURCHFLUSSTESTS
SYST ME D' TALONNAGE INTERNE POUR ESSAI EN COULEMENT CONTINU

(30) Priority: 24.12.2001 US 35014; 25.04.2002 US 132673
(43) Date of publication of application: 22.09.2004
(73) Proprietor: KIMBERLY-CLARK WORLDWIDE, INC., Neenah, WI 54956 (US)
(72) Inventor: WEI, Ning, Roswell, GA 30075 (US); SONG, Xuedong, Roswell, GA 30076 (US)
(74) Representative: Davies, Christopher Robert
(86) International application number: PCT/US2002/037652
(87) International publication number: WO 2003/058242

(56) References cited:
- WO-A-97/09620
- WO-A-99/36777

## Description

### Background of the Invention

Various analytical procedures and devices are commonly employed in flow-through assays to determine the presence and/or concentration of analytes that may be present in a test sample. For instance, immunoassays utilize mechanisms of the immune systems, wherein antibodies are produced in response to the presence of antigens that are pathogenic or foreign to the organisms. These antibodies and antigens, i.e., immunoreactants, are capable of binding with one another, thereby causing a highly specific reaction mechanism that can be used to determine the presence or concentration of that particular antigen in a biological sample.

There are several well-known immunoassay methods that use immunoreactants labeled with a detectable component so that the analyte can be detected analytically. For example, "sandwich-type" assays typically involve mixing the test sample with antibodies to the analyte. These antibodies are mobile and linked to a label or probe, such as dyed latex, a colloidal metal sol, or a radioisotope. This mixture is then contacted with a chromatographic medium containing a band or zone of immobilized antibodies to the analyte. The chromatographic medium is often in the form of a strip resembling a dipstick. When the complex of the analyte and the labeled antibody reaches the zone of the immobilized antibodies on the chromatographic medium, binding occurs and the bound labeled antibodies are localized at the zone. This indicates the presence of the analyte. This technique can be used to obtain quantitative or semi-quantitative results. Some examples of such sandwich-type assays are described in. by U.S. Patent Nos. 4,168,146 to Grubb, et al. and 4,366,241 to Tom, et al.

WO-A-99-36777 discloses a method using a new calibrator and a device and test kit including the calibrator. WO-A-97-09620 discloses a method and apparatus for quantitative and semi-quantitative determination of analyte.

An alternative technique is the "competitive-type" assay. In a "competitive-type" assay, the label is typically a labeled analyte or analyte-analogue that competes for binding of an antibody with any unlabeled analyte present in the sample. Competitive assays are typically used for detection of analytes such as haptens, each hapten being monovalent and capable of binding only one antibody molecule. Examples of competitive immunoassay devices are described in U.S. Patent Nos. 4,235,601 to Deutsch, et al., 4,442,204 to Liotta, and 5,208,535 to Buechler, et al.

Many of these assays rely upon calibration to provide valid and meaningful results, particularly for semi-quantitative and quantitative detections. Specifically, either external or internal calibration systems are generally employed. In an external calibration system, a standard curve is usually obtained from standard samples containing a series of a known amount of analyte, and the results obtained from the samples are then compared with the standard curve to extract the presence and/or amount of the analyte in the sample. The external calibration method is relatively easy to design and simple to implement. However, it is often subject to interference from environmental and batch-to-batch variations, and is thus unreliable.

Conventional internal calibration systems, on the other hand, typically utilize a membrane that has a calibration zone and a detection zone on which the capturing reagent specific for the analyte is immobilized. Unfortunately, the ability of the calibration zone to provide a reliable and accurate comparison to the detection zone is often limited. Moreover, most internal calibration zones are relatively expensive, thereby making them impractical for certain applications.

As such, a need currently exists for an accurate calibration system for flow-through assays that is readily controllable and inexpensive.

### Summary of the Invention

In accordance with the invention, there is provided a flow-through assay as claimed in claim 1.

In accordance with one embodiment of the present invention, a flow-through assay (e.g., sandwich, competitive, etc.) is disclosed for detecting the presence or quantity of an analyte residing in a test sample. The assay comprises a porous membrane that is in fluid communication with a probe conjugate that contains a specific binding member and a detectable probe. For example, in some embodiments, the detectable probe is selected from the group consisting of chromogens, catalysts, fluorescent compounds, chemiluminescent compounds, radioactive labels, direct visual labels, liposomes, and combinations thereof. In one particular embodiment, the detectable probe comprises a latex microparticle.

The porous membrane also defines a detection zone that contains a capture reagent capable of binding to the analyte or the probe conjugate. In some embodiments, for example, the capture reagent is selected from the group consisting of antigens, haptens, antibodies, and complexes thereof. The detection zone is capable of generating a detection signal to indicate the presence or absence of an analyte.

In addition, to assist in the determination of the amount of analyte present within the test sample, the porous membrane also defines a calibration zone that contains a binder configured to bind with the probe conjugate. The calibration zone includes:
i) a first calibration region (e.g., line, dot, etc.) containing a first predetermined amount of the binder, the first calibration region being capable of generating a first calibration signal; and
ii) a second calibration region (e.g., line, dot, etc.) containing a second predetermined amount of the binder that is greater than the first predetermined amount of the binder, the second calibration region being capable of generating a second calibration signal, the second calibration signal having a greater intensity than the first calibration signal.

Once the calibration regions generate signals, they can then be compared to the detection signal to determine the presence or relative amount of analyte in the test sample. For example, in some embodiments, the calibration signals can be visually observed and compared to the detection signal. Moreover, the calibration signals can also be compared to the detection signal through the use of an instrument, such as a fluorescent reader, a color intensity reader, and the like. If desired, a calibration curve can be developed by plotting the intensity of the calibration signals versus known amounts of the analyte. Once generated, the curve can then be used to determine an unknown amount of the analyte within a test sample.

To provide a greater degree of calibration accuracy, the calibration zone can employ more than two calibration regions. For instance, in some embodiments, the calibration zone further includes a third calibration region (e.g., line, dot, etc.) containing a third predetermined amount of the binder that is greater than the second predetermined amount of the binder. The third calibration region is capable of generating a third calibration signal that has a greater intensity than the second calibration signal. It should be understood, however, that any number of calibration regions, such as four or five, may also be used in the present invention.

The geometric disposition of the calibration regions can also be selected to increase or decrease the time required for calibration. For example, in one embodiment, at least one of the calibration regions is disposed in a direction that is substantially perpendicular to the flow of the test sample through the porous membrane. Moreover, in another embodiment, at least one of the calibration regions is disposed in a direction that is substantially parallel to the flow of the test sample through the porous membrane. Such a parallel disposition can allow simultaneous calibration of multiple calibration regions.

Other features and aspects of the present invention are discussed in greater detail below.

### Brief Description of the Drawing

A full and enabling disclosure of the present invention, including the best mode thereof, directed to one of ordinary skill in the art, is set forth more particularly in the remainder of the specification, which makes reference to the appended figures in which:
Fig. 1 is a top view of one embodiment of the present invention, showing a flow-through assay having three calibration lines in a calibration zone;
Fig. 2 is a perspective schematic view of one embodiment of a flow-through assay of the present invention, showing the membrane strip after a test sample containing analyte has been applied to the sampling pad;
Fig. 3 illustrates the lateral assay shown in Fig. 2, but with the test sample migrated through the assay;
Fig. 4 is a top view of another embodiment of the present invention, in which Fig. 4A shows calibration lines substantially parallel to the flow of the analyte and Fig. 4B shows calibration dots substantially parallel to the flow of the analyte;
Fig. 5 shows a calibration curve that may be used in one embodiment of the present invention;
Fig. 6 shows a calibration curve for CRP detection as discussed in Example 3;
Fig. 7 shows a calibration curve for LH detection as discussed in Example 4; and
Fig. 8 shows a calibration curve for pre-albumin detection as discussed in Example 5.

Repeat use of reference characters in the present specification and drawings is intended to represent same or analogous features or elements of the invention.

### Detailed Description of Representative Embodiments

### Definitions

As used herein, the term "analyte" generally refers to a substance to be detected. For instance, analytes can includes antigenic substances, haptens, antibodies, and combinations thereof. Analytes include, but are not limited to, toxins, organic compounds, proteins, peptides, microorganisms, amino acids, nucleic acids, hormones, steroids, vitamins, drugs (including those administered for therapeutic purposes as well as those administered for illicit purposes), bacteria, virus particles and metabolites of or antibodies to any of the above substances. Specific examples of some analytes include ferritin; creatinine kinase MIB (CK-MB); digoxin; phenytoin; phenobarbitol; carbamazepine; vancomycin; gentamycin; theophylline; valproic acid; quinidine; leutinizing hormone (LH); follicle stimulating hormone (FSH); estradiol, progesterone; IgE antibodies; vitamin B2 micro-globulin; glycated hemoglobin (Gly. Hb); cortisol; digitoxin; N-acetylprocainamide (NAPA); procainamide; antibodies to rubella, such as rubella-IgG and rubella IgM; antibodies to toxoplasmosis, such as toxoplasmosis IgG (Toxo-IgG) and toxoplasmosis IgM (Toxo-IgM); testosterone; salicylates; acetaminophen; hepatitis B virus surface antigen (HBsAg); antibodies to hepatitis B core antigen, such as anti-hepatitis B core antigen IgG and IgM (Anti-HBC); human immune deficiency virus 1 and 2 (HIV 1 and 2); human T-cell leukemia virus 1 and 2 (HTLV); hepatitis B e antigen (HBeAg); antibodies to hepatitis B e antigen (Anti-HBe); thyroid stimulating hormone (TSH); thyroxine (T4); total triiodothyronine (Total T3); free triiodothyronine (Free T3); carcinoembryoic antigen (CEA); and alpha fetal protein (AFP). Drugs of abuse and controlled substances include, but are not intended to be limited to, amphetamine; methamphetamine; barbiturates, such as amobarbital, secobarbital, pentobarbital, phenobarbital, and barbital; benzodiazepines, such as librium and valium; cannabinoids, such as hashish and marijuana; cocaine; fentanyl; LSD; methaqualone; opiates, such as heroin, morphine, codeine, hydromorphone, hydrocodone, methadone, oxycodone, oxymorphone and opium; phencyclidine; and propoxyhene. Other potential analytes may be described in U.S. Patent No. 4,366,241 to Tom et al.

As used herein, the term "test sample" generally refers to a material suspected of containing the analyte. The test sample can be used directly as obtained from the source or following a pretreatment to modify the character of the sample. The test sample can be derived from any biological source, such as a physiological fluid, including, blood, saliva, ocular lens fluid, cerebral spinal fluid, sweat, urine, milk, ascites fluid, raucous, synovial fluid, peritoneal fluid, amniotic fluid or the like. The test sample can be pretreated prior to use, such as preparing plasma from blood, diluting viscous fluids, and the like. Methods of treatment can involve filtration, distillation, concentration, inactivation of interfering components, and the addition of reagents. Besides physiological fluids, other liquid samples can be used such as water, food products and the like for the performance of environmental or food production assays. In addition, a solid material suspected of containing the analyte can be used as the test sample. In some instances it may be beneficial to modify a solid test sample to form a liquid medium or to release the analyte.

### Detailed Description

Reference now will be made in detail to various embodiments of the invention, one or more examples of which are set forth below. Each example is provided by way of explanation of the invention, not limitation of the invention. In fact, it will be apparent to those skilled in the art that various modifications and variations can be made in the present invention without departing from the scope of the invention. For instance, features illustrated or described as part of one embodiment, can be used on another embodiment to yield a still further embodiment. Thus, it is intended that the present invention covers such modifications and variations as come within the scope of the appended claims and their equivalents.

In general, the present invention is directed to an internal calibration system for flow-through assays. In particular, the present invention employs the use of a calibration zone that contains two or more distinct calibration regions (e.g., lines, dots, etc.). The calibration regions contain a different amount of a binder so that one region is capable of generating a calibration signal that is less intense than the calibration signal generated by the other regions. In one embodiment, a calibration curve can be developed for the level of binder in each calibration region for comparison to a detection signal. It has been discovered that the internal calibration system provides an accurate, inexpensive, and readily controllable method of determining the presence of an analyte in a test sample.

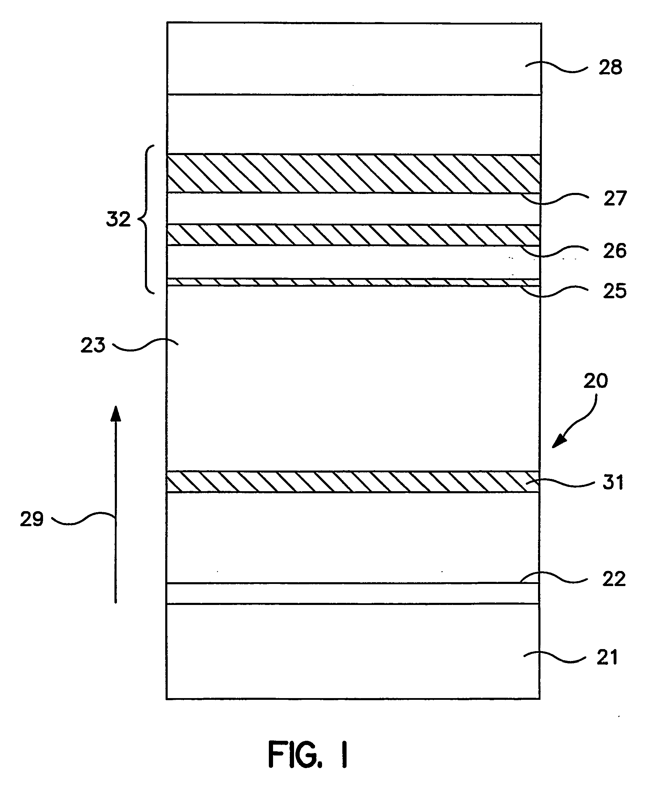

Referring to Figs. 1-3, for instance, one embodiment of a sandwich-type flow-through assay 20 that can be formed according to the present invention will now be described in more detail. As shown, the assay 20 is contains a porous membrane 23 optionally supported by a rigid material (not shown). In general, the porous membrane 23 can be made from any of a variety of materials through which the test sample is capable of passing. For example, the materials used to form the porous membrane 23 can include, but are not limited to, natural, synthetic, or naturally occurring materials that are synthetically modified, such as polysaccharides (e.g., cellulose materials such as paper and cellulose derivatives, such as cellulose acetate and nitrocellulose); silica; inorganic materials, such as deactivated alumina, diatomaceous earth, MgSO₄, or other inorganic finely divided material uniformly dispersed in a porous polymer matrix, with polymers such as vinyl chloride, vinyl chloride-propylene copolymer, and vinyl chloride-vinyl acetate copolymer; cloth, both naturally occurring (e.g., cotton) and synthetic (e.g., nylon or rayon); porous gels, such as silica gel, agarose, dextran, and gelatin; polymeric films, such as polyacrylamide; and the like. In one particular embodiment, the porous membrane 23 is formed from nitrocellulose and/or polyester sulfone materials. It should be understood that the term "nitrocellulose" refers to nitric acid esters of cellulose, which may be nitrocellulose alone, or a mixed ester of nitric acid and other acids, such as aliphatic carboxylic acids having from 1 to 7 carbon atoms.

To initiate the detection of an analyte 40 within the test sample, a user may directly apply the test sample to a portion of the porous membrane 23 through which it can then travel to reach one or more detection and calibration zones (described below). Alternatively, the test sample may first be applied to a sampling pad that is in fluid communication with the porous membrane 23. For example, as shown in Figs. 1-3, the lateral flow assay 20 can contain a sampling pad 21 generally configured to receive the test sample. Some suitable materials that can be used to form the sampling pad 21 include, but are not limited to, nitrocellulose, cellulose, porous polyethylene pads, and glass fiber filter paper. If desired, the sampling pad 21 may also contain one or more assay pretreatment reagents, either diffusively or non-diffusively attached thereto.

In the illustrated embodiment, the test sample travels from the sampling pad 21 to a conjugate pad 22 (as shown by the directional arrow 29 in Fig. 1) that is placed in communication with one end of the sampling pad 21. The conjugate pad 22 is formed from a material through which the test sample is capable of passing. For example, in one embodiment, the conjugate pad 22 is formed from glass fibers.

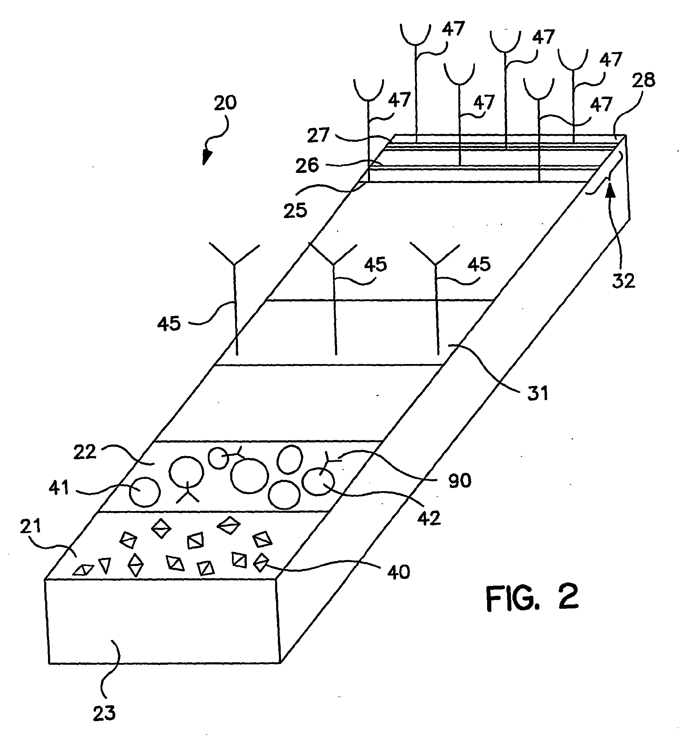

Besides simply allowing the test sample to pass therethrough, the conjugate pad 22 also typically performs other functions as well. For example, in some embodiments, various probes 41 (see Fig. 2) are releasibly applied to the conjugate pad 22. While contained on the conjugate pad 22, these probes 41 remain available for binding with the analyte 40 as the analyte 40 passes from the sample pad 21 through the conjugate pad 22. Upon binding with the analyte 40, the probes 41 can later serve to identify (e.g., visually, etc.) the presence of the analyte 40 in the detection zone of the assay 20.

Any substance generally capable of producing a signal that is visually detectable or detectable by an instrumental device may be used as the probes 41. Various suitable probes can include chromogens; catalysts; fluorescent compounds; chemiluminescent compounds; radioactive labels; direct visual labels, including colloidal metallic and non-metallic particles (e.g., gold), dye particles, enzymes or substrates, or organic polymer latex particles; liposomes or other vesicles containing signal producing substances; and the like. For instance, some enzymes suitable for use as probes are disclosed in U.S. Patent No. 4,275,149 to Litman, et al. One example of an enzyme/substrate probe system is the enzyme alkaline phosphatase and the substrate nitro blue tetrazolium-5-bromo-4-chloro-3-indolyl phosphate, or derivative or analog thereof, or the substrate 4-methylumbelliferyl-phosphate. In an alternative probe system, the probe can be a fluorescent compound where no enzymatic manipulation is required to produce a detectable signal. Fluorescent molecules, such as fluorescein, phycobiliprotein, rhodamine and their derivatives and analogs, are suitable for use as probes in this reaction. Commercially available examples of such fluorescent materials include fluorescent carboxylated microspheres sold by Molecular Probes, Inc. under the trade names "FluoSphere" (Red 580/605) and "TransfluoSphere" (543/620), as well as "Texas Red" and 5- and 6-carboxytetramethylrhodamine, which are also sold by Molecular Probes, Inc.

A visually detectable, colored microparticle (sometimes referred to as "beads" or "microbeads") can also be used as a probe, thereby providing for a direct colored readout of the presence or concentration of the analyte in the sample without the need for further signal producing reagents. In some instances, the particles that are used in a quantitative assay can also contribute a signal (e.g., light absorption) that would cause the zone in which the particles are located to have a different signal than the rest of the membrane 23.

The type of microparticles utilized for the probes 41 may also vary. For instance, naturally occurring microparticles, such as nuclei, mycoplasma, plasmids, plastids, mammalian cells (e.g., erythrocyte ghosts), unicellular microorganisms (e.g., bacteria), polysaccharides (e.g., agarose), and the like, can be used. Further, synthetic microparticles may also be utilized. For example, in one embodiment, synthetic latex microparticles that are colored with a dye are utilized as the probes 41. Although any latex microparticle capable of adsorbing or covalently bonding to a binding partner may be used in the present invention, the latex microparticles are typically formed from polystyrene, butadiene styrenes, styreneacrylic-vinyl terpolymer, polymethylmethacrylate, polyethylmethacrylate, styrene-maleic anhydride copolymer, polyvinyl acetate, polyvinylpyridine, polydivinylbenzene, polybutyleneterephthalate, acrylonitrile, vinylchloride-acrylates, and the like, or an aldehyde, carboxyl, amino, hydroxyl, or hydrazide derivative thereof. Other suitable microparticles may be described in U.S. Patent Nos. 5,670,381 to Jou, et al. and 5,252,459 to Tarcha, et al. Commercially available examples of suitable colored, latex microparticles include carboxylated latex beads sold by Bang's Laboratory, Inc.

When utilized, the mean diameter of particulate probes 41 may generally vary as desired depending on factors such as the type of particle chosen, the pore size of the membrane, and the membrane composition. For example, in some embodiments, the mean diameter of the particulate probes 41 ranges from about 0.01 microns to about 100 microns, and in some embodiments, from about 0.1 microns to about 75 microns. In one particular embodiment, the particulate probes 41 have a mean diameter of about 0.3 microns. In such instances, the membrane 23 can have a pore size of from about 0.1 to about 0.3 microns.

When deposited on the conjugate pad 22, the probes 41 may be capable of directly bonding (covalently or non-covalently) with the analyte 40. However, it is often desired to modify the probes 41 in some manner so that they are more readily able to bond to the analyte 40. In such instances, the probes 41 can be modified with certain specific binding members 90 that are non-covalently (e.g., adsorbed) and/or covalently attached thereto to form probe conjugates 42.

Specific binding members generally refer to a member of a specific binding pair, i.e., two different molecules where one of the molecules chemically and/or physically binds to the second molecule. For instance, immunoreactive specific binding members can include antigens, haptens, antibodies, and complexes thereof, including those formed by recombinant DNA methods or peptide synthesis. An antibody can be a monoclonal or polyclonal antibody, a recombinant protein or a mixture(s) or fragment(s) thereof, as well as a mixture of an antibody and other specific binding members. The details of the preparation of such antibodies and their suitability for use as specific binding members are well known to those skilled in the art.

Other common specific binding pairs include but are not limited to, biotin and avidin, carbohydrates and lectins, complementary nucleotide sequences (including probe and capture nucleic acid sequences used in DNA hybridization assays to detect a target nucleic acid sequence), complementary peptide sequences including those formed by recombinant methods, effector and receptor molecules, hormone and hormone binding protein, enzyme cofactors and enzymes, enzyme inhibitors and enzymes, and the like. Furthermore, specific binding pairs can include members that are analogs of the original specific binding member. For example, a derivative or fragment of the analyte, i.e., an analyte-analog, can be used so long as it has at least one epitope in common with the analyte.

The specific binding members 90 can generally be attached to the probes 41 using any of a variety of well-known techniques. For instance, when using latex microparticles as the probes 41, covalent attachment of the specific binding members 90 thereto can be accomplished using carboxylic, amino, aldehyde, bromoacetyl, iodoacetyl, thiol, epoxy and other reactive or linking functional groups, as well as residual free radicals and radical cations, through which a protein coupling reaction can be accomplished. A surface functional group can also be incorporated as a functionalized co-monomer because the surface of the latex microparticle can contain a relatively high surface concentration of polar groups. In addition, although latex microparticle probes are typically functionalized after synthesis, in certain cases, such as poly(thiophenol), the microparticles are capable of direct covalent linking with a protein without the need for further modification.

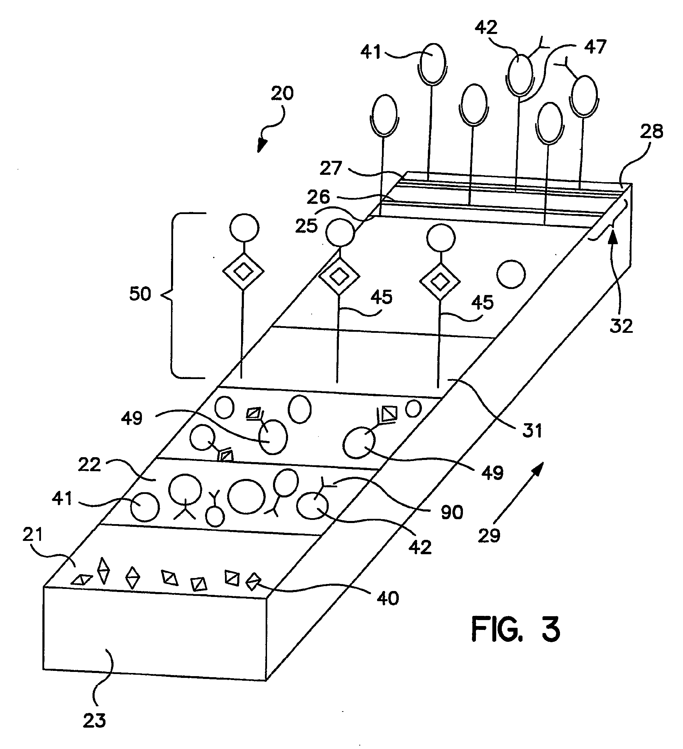

Thus, referring again to Figs. 2 and 3, a test sample containing an analyte 40 can initially be applied to the sampling pad 21. From the sampling pad, the test sample can then travel to the conjugate pad 22, where the analyte 40 binds to the specific binding member 90 of a probe conjugate 42 to form a probe conjugate/analyte complex 49. Moreover, because the conjugate pad 22 is in fluid communication with the porous membrane 23, the probe conjugate/analyte complex 49 can migrate from the conjugate pad 22 to a detection zone 31 present on the porous membrane 23.

The detection zone 31 may contain an immobilized capture reagent 45. Although not required, it may be desired that the capture reagents 45 be formed from the same class or category of materials (e.g., antibodies) as the specific binding members 90 used to form the probe conjugates 42. These capture reagents 45 serve as stationary binding sites for the probe conjugate/analyte complexes 49. In some instances, the analytes 40, such as antibodies, antigens, etc., have two binding sites. Upon reaching the detection zone 31, one of these binding sites is occupied by the specific binding member 90 of the probe conjugate/analyte complex 49. However, the free binding site of the analyte 40 can bind to the immobilized capture reagent 45. Upon being bound to the immobilized capture reagent 45, the probe conjugate 42 of a newly formed ternary complex 50 signals the presence of the analyte 40, either visually or through other methods of detection (e.g., instruments, etc.). Thus, to determine whether a particular analyte 40 is present within a test sample, a user can simply analyze the detection zone 31.

However, although a detection zone may indicate the presence of an analyte, it is often difficult to determine the relative concentration of the analyte within a test sample using solely a detection zone. Thus, in accordance with the present invention, the assay also includes a calibration zone that may be compared to the detection zone for determining the concentration of a particular analyte within a test sample. For instance, referring again to Figs. 1-3, one embodiment of a flow-through assay 20 that includes a calibration zone 32 is illustrated. In this embodiment, the calibration zone 32 is formed on the porous membrane and is positioned downstream from the detection zone 31. The control zone 32 is provided with a binder 47 that is capable of binding to any remaining probes 41 and/or probe conjugates 42 that pass through the length of the membrane 23. In particular, upon being contacted with the test sample, any probes 41 and/or probe conjugates 42 that do not bind to the analyte 40 migrate through the detection zone 31 with the complexes 49. In the detection zone 31, as set forth above, the complexes 49 bind to capture reagents 45 and remain immobilized. However, the unbound probes 41 and/or probe conjugates 42 continue to migrate through the detection zone 31 and enter the calibration zone 32 of the porous membrane 23. At the calibration zone 32, these unbound probes 41 and/or probe conjugates 42 then bind to the binders 47. When immobilized in the calibration zone 32, the probes 41 and/or probe conjugates 42 are observable, either visually or by other methods, so that a user can compare the signal intensity in the detection zone 31 to the signal intensity in the calibration zone 32.

The calibration zone 32 may generally provide any number of distinct calibration regions so that a user can better determine the concentration of a particular, analyte within a test sample. In most embodiments, for example, the calibration zone 32 includes two or more calibration distinct calibration regions (e.g., lines, dots, etc.). For instance, in the illustrated embodiment, at least three calibration regions 25, 26, and 27 in the form of lines are utilized. As shown in Figs. 1-3, the calibration regions 25, 26, and/or 27 may be disposed in the form of lines in a direction that is substantially perpendicular to the flow of the test sample through the assay 20.

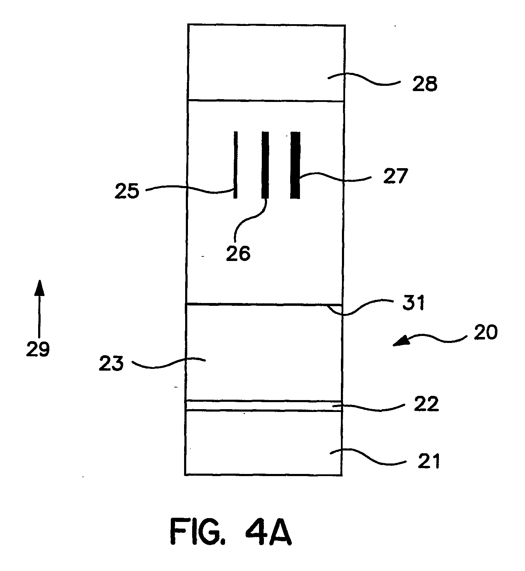

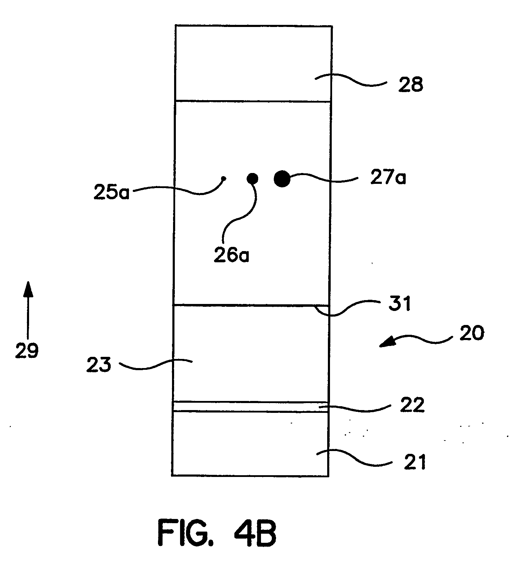

Likewise, in some embodiments, such as shown in Fig. 4A, the calibration regions 25, 26, and/or 27 can be disposed in the form of lines in a direction that is substantially parallel to the flow of the test sample through the assay. In yet another embodiment, such as shown in Fig. 4B, three calibration regions 25a, 26a, and 27a are disposed in the form of dots in a direction that is substantially parallel to the flow of the test sample through the assay. In such instances, a user may be able to compare the calibration signal to the detection signal in a lesser amount of time because each of the calibration regions simultaneously generate a calibration signal.

The calibration regions 25, 26, and 27 may be pre-loaded on the porous membrane 23 with different amounts of the binder 47 so that a different signal intensity is generated by each calibration region 25, 26, and 27 upon migration of the probes 41 and/or probe conjugates 42. The overall amount of binder 47 within each calibration region can be varied by utilizing calibration regions of different sizes and/or by varying the solution concentration or volume of the binder 47 in each calibration region. Generally speaking, the concentration of a binder 47 within a given calibration region can range from about 0.01% to about 25% by weight of the solution.

If desired, an excess of probe molecules can be employed in the assay 20 so that each calibration region 25, 26, and 27 reaches its full and predetermined potential for signal intensity. That is, the amount of probes 41 that are deposited upon calibration regions 25, 26, and 27 are predetermined because the amount of the binder 47 employed on the calibration regions 25, 26, and 27 is set at a predetermined and known level. A comparison may be made between the intensity levels of the calibration regions 25, 26, and 27 and the detection line 24 to calculate the amount of analyte 40 present in the test sample. This comparison step may occur visually, with the aid of a reading device, or using other techniques.

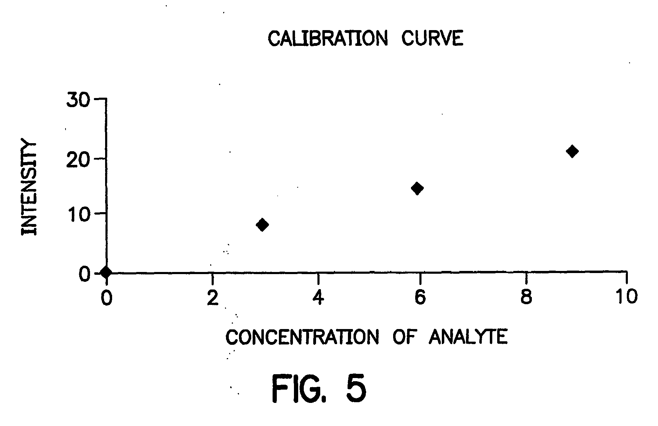

Calibration and sample testing may be conducted under approximately the same conditions at the same time, thus providing reliable quantitative results, with increased sensitivity. The assay 20 may also be employed for semi-quantitative detection. Specifically, when multiple calibration regions 25, 26, and 27 provide a range of signal intensities, the signal intensity of the detection zone 31 can be compared (e.g., visually) with the intensity of the calibration regions 25, 26, and 27. Based upon the intensity range in which the detection zone 31 falls, the general concentration range for the analyte 40 may be determined. If desired, the signal ratio between the detection zone 31 and the calibration regions 25, 26, and 27 may be plotted versus analyte concentration for a range of known analyte concentrations to generate a calibration curve, such as shown in Fig. 5. To determine the quantity of an unknown test sample, the signal ratio may then be converted to analyte concentration according to the calibration curve. Moreover, when using fluorescence to determine the amount of analyte 40 in a test sample, a receiver or a receiving device can be used to measure the amount of fluorescence generated in the detection zone 31 and the calibration zone 32, and thereafter make the appropriate comparison to determine the quantity of analyte in a given test sample.

The binders 47 utilized in the calibration zone 32 can generally be formed from a variety of different materials capable of forming a chemical or physical bond with the probes 41 and/or probe conjugates 42. For example, in some embodiments, the binders 47 can contain a biological capture reagent that is the same or different than the capture reagents 45. Such biological capture reagents are well known in the art and can include, but are not limited to, antigens, haptens, antibodies, and complexes thereof.

In addition, it may also be desired to utilize various non-biological materials for the binders 47. The binders 47 are a polyelectrolyte that can bind to the probes 41 and/or probe conjugates 42. The polyelectrolytes can have a net positive or negative charge, as well as a net charge that is generally neutral. For instance, some suitable examples of polyelectrolytes having a net positive charge include, but are not limited to, polylysine (commercially available from Sigma-Aldrich Chemical Co., Inc. of St. Louis, MO), polyethylenimine; epichlorohydrin-functionalized polyamines and/or polyamidoamines, such as poly(dimethylamine-co-epichlorohydrin); polydiallyldimethyl-ammonium chloride; cationic cellulose derivatives, such as cellulose copolymers or cellulose derivatives grafted with a quaternary ammonium water-soluble monomer, and the like. In one particular embodiment, CelQuat® SC-230M or H-100 (available from National Starch & Chemical, Inc.), which are cellulosic derivatives containing a quaternary ammonium water-soluble monomer, can be utilized. Moreover, some suitable examples of polyelectrolytes having a net negative charge include, but are not limited to, polyacrylic acids, such as poly(ethylene-co-methacrylic acid, sodium salt), and the like. It should also be understood that other polyelectrolytes may also be utilized in the present invention, such as amphiphilic polyelectrolytes (i.e., having polar an non-polar portions). For instance, some examples of suitable amphiphilic polyelectrolytes include, but are not limited to, poly(styryl-b-N-methyl 2-vinyl pyridinium iodide) and poly(styryl-b-acrylic acid), both of which are available from Polymer Source, Inc. of Dorval, Canada.

Although any polyelectrolyte may generally be used, the polyelectrolyte selected for a particular application may vary depending on the nature of the probes/probe conjugates, the porous membrane, and the like. In particular, the distributed charge of a polyelectrolyte allows it to bind to substances having an opposite charge. Thus, for example, polyelectrolytes having a net positive charge are often better equipped to bind with probes 41 and/or probe conjugates 42 that are negatively charged, while polyelectrolytes that have a net negative charge are often better equipped to bind to probes 41 and/or probe conjugates 42 that are positively charged. Thus, in such instances, the ionic interaction between these molecules allows the required binding to occur within the calibration zone 32. Nevertheless, although ionic interaction is primarily utilized to achieve the desired binding in the calibration zone 32, it has also been discovered that polyelectrolytes can also bind with probes 41 and/or probe conjugates 42 having a similar charge.

Because the polyelectrolyte is designed to bind to the probes 41 and/or probe conjugates 42 to provide a calibration signal, it is typically desired that the polyelectrolyte be substantially non-diffusively immobilized on the surface of the porous membrane 23. Otherwise, the probes 41 and/or probe conjugates 42 would not be readily detectable by a user seeking to calibrate the assay. Thus, the polyelectrolytes can be applied to the porous membrane 23 in such a manner that the polyelectrolytes do not substantially diffuse into the matrix of the porous membrane 23. In particular, the polyelectrolytes typically form an ionic and/or covalent bond with functional groups present on the surface of the porous membrane 23 so that they remain immobilized thereon. Although not required, the formation of covalent bonds between the polyelectrolyte and the porous membrane 23 may be desired to more permanently immobilize the polyelectrolyte thereon.

For example, in one embodiment, the monomers used to form the polyelectrolyte are first formed into a solution and then applied directly to the porous membrane 23. Various solvents (e.g., organic solvents, water, etc.) may be utilized to form the solution. Once applied, the polymerization of the monomers is initiated using heat, electron beam radiation, free radical polymerization, and the like. In some instances, as the monomers polymerize, they form covalent bonds with certain functional groups of the porous membrane 23, thereby immobilizing the resulting polyelectrolyte thereon. For example, in one embodiment, an ethyleneimine monomer can form a covalent bond with a carboxyl group present on the surface of some porous membranes (e.g., nitrocellulose).

In another embodiment, the polyelectrolyte can be formed prior to application to the porous membrane 23. If desired, the polyelectrolyte may first be formed into a solution using organic solvents, water, and the like. Thereafter, the polyelectrolytic solution is applied directly to the porous membrane 23 and then dried. Upon drying, the polyelectrolyte may, as described above, form an ionic bond with certain functional groups present on the surface of the porous membrane 23 that have a charge opposite to the polyelectrolyte. For example, in one embodiment, positively-charged polyethyleneimine can form an ionic bond with negatively-charged carboxyl groups present on the surface of some porous membranes (e.g., nitrocellulose).

In addition, the polyelectrolyte may also be crosslinked to the porous membrane 23 using various well-known techniques. For example, in some embodiments, epichlorohydrin-functionalized polyamines and/or polyamidoamines can be used as a crosslinkable, positively-charged polyelectrolyte. Examples of these materials are described in U.S. Pat. Nos. 3,700,623 to Keim and 3,772,076 to Keim, 4,537,657 to Keim, believed to be sold by Hercules, Inc., Wilmington, Del. under the Kymene^{™} trade designation. For instance, Kymene^{™} 450 and 2064 are epichlorohydrin-functionalized polyamines and/or polyamidoamines that contain epoxide rings and quaternary ammonium groups that can form covalent bonds with carboxyl groups present on certain types of porous membranes (e.g., nitrocellulose) and crosslink with the polymer backbone of the porous membrane when cured. In some embodiments, the crosslinking temperature can range from about 50°C to about 120°C and the crosslinking time can range from about 10 to about 600 seconds.

Although various techniques for non-diffusively immobilizing polyelectrolytes on the porous membrane 23 have been described above, it should be understood that any other technique for non-diffusively immobilizing polyelectrolytic compounds can be used in the present invention. In fact, the aforementioned methods are only intended to be exemplary of the techniques that can be used in the present invention. For example, in some embodiments, certain components may be added to the polyelectrolyte solution that can substantially inhibit the diffusion of such polyelectrolytes into the matrix of the porous membrane 23.

Beside the above-mentioned components, the flow-through assay 20 may also contain additional components. For example, referring again to Figs. 1-3, the assay 20 can also contain a wicking pad 28. The wicking pad 28 generally receives fluid that has migrated through the entire porous membrane 23. As is well known iri the art, the wicking pad 28 can assist in promoting capillary action and fluid flow through the membrane 23.

Although various embodiments of assay configurations have been described above, it should be understood, that an assay of the present invention may generally have any configuration desired, and need not contain all of the components described above. Further, other well-known components of assays not specifically referred to herein may also be utilized in the present invention. For example, various assay configuration are described in U.S. Patent Nos. 5,395,754 to Lambotte, et al.; 5,670,381 to Jou, et al.; and 6,194,220 to Malick et al. In addition, it should also be understood that competitive assays may also be formed according to the present invention. Techniques and configurations of competitive assays are well known to those skilled in the art.

For instance, in one embodiment, the flow-through assay 20 described above and illustrated in Figs. 1-3 can be easily modified to form a competitive assay by utilizing probe conjugates 42 that contain specific binding members 90 identical to the analyte 40. As a result, the analyte 40 and probe conjugates 42 will compete for a predetermined number of capture reagents 45 in the detection zone 31. Generally speaking, because the analyte 40 is unbound, it will move faster through the porous membrane and occupy a greater number of binding sites in the detection zone 31. Any unbound probe conjugates 42 will then travel to the calibration zone 32 where they can bind with the binder 47. The signal thus generated in the calibration zone 32 can be compared to the signal generated in the detection zone 31, wherein the relative amount of analyte in the test sample is inversely proportional to the intensity of the detection signal and directly proportional to the intensity of the calibration signal.

Likewise, in another embodiment, a competitive assay can be formed by utilizing capture reagents 45 that are identical to the analyte 40. Thus, in this embodiment, the probe conjugates 42 initially bind to the analyte 40 to form ternary complexes 49. The unbound probe conjugates 42 and ternary complexes 49 then migrate to the detection zone 31, where the unbound probe conjugates 42 bind to the capture reagent 45. Any remaining unbound probe conjugates 42 and the ternary complexes 49 will then migrate to the calibration zone 32, where they compete for a predetermined amount of the binder 47. The signal thus generated in the calibration zone 32 can be compared to the signal generated in the detection zone 31, wherein the relative amount of analyte in the test sample is inversely proportional to the intensity of the detection signal and directly proportional to the intensity of the calibration signal.

The present invention may be better understood with reference to the following examples.

### EXAMPLE 1

The ability of an internal calibration zone of the present invention to calibrate a sandwich assay was demonstrated. Initially, Millipore SX porous membrane samples made of nitrocellulose were laminated onto corresponding supporting cards having a length of approximately 30 centimeters. Aqueous solutions of polyethylenimine were then stripped onto the membrane (1x, 10x, and 100x dilution of 7.4% polyethyleneimine solution) to form three separate calibration lines of different concentrations. After application of the polyethylenimine, the membranes were dried for 1 hour at a temperature of 37°C.

A cellulosic fiber wicking pad (Millipore Co.) was attached to one end of the membrane. The other end of the membrane was inserted into a variety of probe and probe conjugate suspensions. In particular, the following probes were tested:

| Probe | Color | Particle Size (microns) | Net Charge | Vendor |
|---|---|---|---|---|
| Colored Carboxylate Latex Beads | Blue | 0.3 | Positive | Bang's Laboratory, Inc. |
| Fluorescent Carboxylate Latex Beads | Red | 0.5 | Positive | Molecular Probes, Inc. |

The assays were also inserted into suspensions of probe conjugates. In particular, the above-mentioned probes were conjugated with anti-C-reactive protein monoclonal antibody (anti-CRP Mab), anti-leutinizing hormone monoclonal antibody (anti-LH Mab), and anti-prealbumin polyclonal antibody (anti-Pab) using well-known techniques. For instance, a 100-microliter suspension of the 0.5-micron fluorescent carboxylated microspheres (available from Molecular Probes, Inc.) was initially washed two times with a phosphate buffer saline (PBS) and then re-suspended in 200 microliters of PBS. To the suspension, 5 mg carbodiimide was added and the mixture was mixed gently for 1 hour. The microspheres were then washed twice with a borate buffer and then washed. The microspheres were re-suspended in a 185-microliter borate buffer. 15 microliters of α-LH monoclonal antibody (9.7 mg/ml) was then added to the suspension and allowed to react for 3 hours under gentle mixing. Thereafter, 200 microliters of a 1M ethanolamine aqueous solution was added to the reaction mixture for 20 minutes. The microspheres were then washed two times using PBS and stored in PBS.

The probe and probe conjugate suspensions contained water and 1.6% polyoxyethylene sorbitan monolaurate (a nonionic surfactant available from Sigma-Aldrich under the name "Tween 20"). The resulting concentration of the probes ranged from 0.001-5 mg/ml and the concentration of the probe conjugates range from 0.2-10 mg/ml.

After about 5 minutes, the stripped calibration lines were then observed to determine if the probes/probes conjugates were visually detectable. The line containing the 1x diluted solution exhibited the highest signal intensity, while the line containing the 100x diluted exhibited the lowest signal intensity.

### EXAMPLE 2

The ability of an internal calibration zone of the present invention to calibrate a half-dipstick sandwich assay was demonstrated. Initially, Millipore SX porous membrane samples made of nitrocellulose were laminated onto corresponding supporting cards having a length of approximately 30 centimeters. 7.4% polyethylenimine aqueous solutions (1 x, 10x, and 100x diluted samples) were then stripped onto the Millipore SX membrane to form three calibration lines of different concentrations.

Anti-C-reactive protein (anti-CRP) monoclonal antibody (Mab A5804, 1 mg/ml, obtained from BiosPacific, Inc.) was stripped onto the membrane to form a detection line. The membrane was dried for 1 hour at a temperature of 37°C. A cellulosic fiber wicking pad (Millipore Co.) was attached to one end of the membrane. The laminated membrane was then cut into small half dipsticks.

The end of the membrane opposite to the wicking pad was applied to a test well that contained C-reactive protein (CRP), Tween 20, anti-CRP Mab conjugated to blue latex beads (anti-CRP Mab-beads), and water. The mixture in the well migrated along the half dipstick to the detection line, calibration lines, and wicking pad of the dipstick.

The CRP analyte was captured by the anti-CRP Mab-beads at the detection line, while any remaining unbound anti-CRP Mab-beads were captured by the calibration lines. Thus, after about 5 minutes, one blue line was observed on the detection line, while three blue lines were observed on the calibration lines. The line containing the 1x diluted solution exhibited the highest signal intensity, while the line containing the 100x diluted exhibited the lowest signal intensity.

### EXAMPLE 3

The ability of an internal calibration zone of the present invention to calibrate a half-dipstick sandwich assay was demonstrated. Initially, HF 09002 porous membrane samples made of nitrocellulose were laminated onto corresponding supporting cards having a length of approximately 30 centimeters. 0.14% (calibration #1), 0.64% (calibration #2), and 1.4% (calibration #3) polyethylenimine aqueous solutions (1x, 10x, and 100x diluted samples) were then stripped onto the membrane to form three calibration lines of different concentrations.

Anti-C-reactive protein (anti-CRP) monoclonal antibody (Mab A5804, 1 mg/ml, obtained from BiosPacific, Inc.) was stripped onto the membrane to form a detection line. The membrane was dried for 1 hour at a temperature of 37°C. A cellulosic fiber wicking pad (Millipore Co.) was attached to one end of the membrane. The laminated membrane was then cut into small half dipsticks.

The end of the membrane opposite to the wicking pad was applied to three test wells that contained Tween 20, an excess amount of anti-CRP Mab conjugated to blue latex beads (anti-CRP Mab-beads), and water. The test wells also contained different concentrations of C-reactive protein (CRP). In particular, the solutions contained 0 nanograms (ng), 0.54 ng, 5.4 ng, and 54 ng of CRP, respectively.

The mixture in the wells migrated along each half dipstick to the detection line, calibration lines, and wicking pad of the dipstick. The CRP analyte was captured by the anti-CRP Mab-beads at the detection line, while any remaining unbound anti-CRP Mab-beads were captured by the calibration lines. Thus, for each sample, one blue line was observed on the detection line, while three blue lines were observed on the calibration lines. The line containing the 1.4% polyethyleneimine solution exhibited the highest signal intensity, while the line containing the 0.14% polyethyleneimine solution exhibited the lowest signal intensity. Based on analysis, it was determined that calibration line #1 contained 0.54 ng of CRP, calibration line #2 contained 5.4 ng of CRP, and calibration line #3 contained 54 ng of CRP.

Thus, when an unknown test sample is tested, CRP concentration can be visually determined by comparing the detection line with the three calibration lines. In particular, when the detection line intensity is visually determined to have an intensity between the intensity of calibration lines #2 and #3, the CRP concentration is between 5.4 and 54 ng. Likewise, when the detection line intensity is visually determined to have an intensity between the intensity of calibration lines #1 and #2, the CRP concentration is between 0.54 and 5.4 ng. Further, a detection line having an intensity less than the intensity of the calibration line #1 has a CRP concentration less than 0.54 ng, while a detection line having an intensity greater than the intensity of the calibration line #3 has a CRP concentration greater than 54 ng.

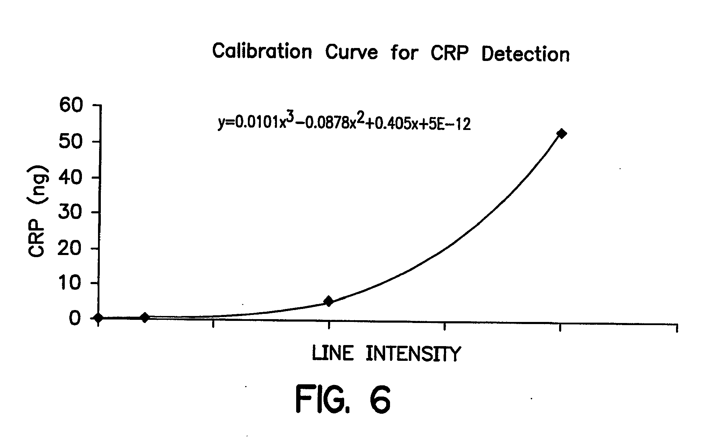

The calibration line intensity can also be measured by an instrument, such as an assay reader. For example, a calibration curve (shown in Fig. 6) was developed using the line intensities of calibration lines #1 - #3 and their CRP concentrations. The mathematical equation generated by the calibration curve can be inputted into an instrument that is able to read intensity for detection of CRP in a test sample.

### EXAMPLE 4

The ability of an internal calibration zone of the present invention to calibrate a half-dipstick sandwich assay was demonstrated. Initially, SHF 075 porous membrane samples made of nitrocellulose were laminated onto corresponding supporting cards having a length of approximately 30 centimeters. Varying concentrations of CelQuat® H-100 (a cellulosic derivative available from National Starch & Chemical, Inc.) were stripped onto the membrane to form three calibration lines having different concentrations. In particular, the concentrations utilized were 2.5 parts CelQuat® H-100 per million of the solution (ppm) (calibration #1), 5 ppm (calibration #2), and 20 ppm (calibration #3).

Anti-β-utilizing hormone (anti-β-LH) monoclonal antibody (Mab, 1mg/ml, obtained from Fitzgerald Industries Intl., Inc.) was stripped onto the membrane to form a detection line. The membrane was dried for 1 hour at a temperature of 37°C. A cellulosic fiber wicking pad (Millipore Co.) was attached to one end of the membrane. The laminated membrane was then cut into small half dipsticks.

The end of the membrane opposite to the wicking pad was applied to a test well that contained Tween 20, anti-α-leutinizing hormone (anti-α-LH) Mab conjugated to blue latex beads (anti-α-LH Mab-beads), and water. The mixture also contained varying concentrations of β-leutinizing hormone (LH). In particular, the concentrations tested were 0 ppm, 20 ppm, and 100 ppm, which corresponded to solutions containing 0 nanograms (ng), 20 ng, and 100 ng of LH, respectively.

The mixture in the wells migrated along each half dipstick to the detection line, calibration lines, and wicking pad of the dipstick. The LH analyte was captured by the anti-α-LH Mab-beads at the detection line, while any remaining unbound anti-α-LH Mab-beads were captured by the calibration lines. Thus, for each sample, one blue line was observed on the detection line, while three blue lines were observed on the calibration lines. The line containing the 20 ppm CelQuat® solution exhibited the highest signal intensity, while the line containing the 2.5 ppm CelQuat® solution exhibited the lowest signal intensity. Based on analysis, it was determined that calibration line #1 contained 20 ng of LH and calibration line #3 contained 100 ng of LH. Moreover, using an instrument capable of reading line intensity, it was determined that calibration lines #1, #2, and #3 had a line intensity of 1, 2, and 4, respectively.

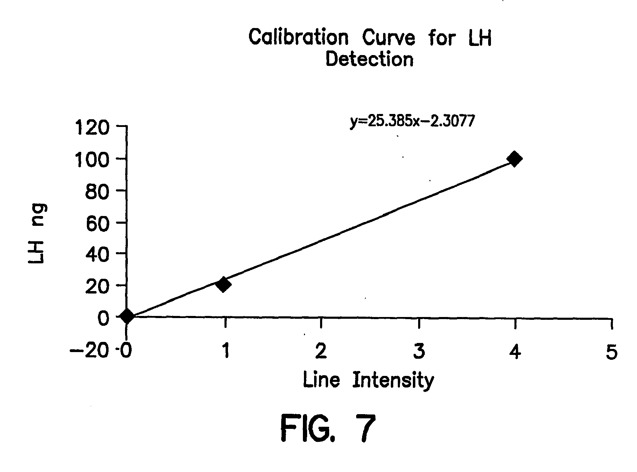

A calibration curve (shown in Fig. 7) was then developed using the line intensities of calibration lines #1- #3 and their LH concentrations. The mathematical equation generated by the calibration curve was then inputted into an instrument. A test sample containing an unknown level of LH was then applied to a membrane formed as described above. Using the instrument, it was determined that the intensity of the detection signal was about 1.5. As a result, it was determined that the concentration of the LH in the unknown test sample was about 36 ng.

### EXAMPLE 5

The ability of an internal calibration zone of the present invention to calibrate a half-dipstick competitive assay was demonstrated. Initially, HF 120 porous membrane samples made of nitrocellulose were laminated onto corresponding supporting cards having a length of approximately 30 centimeters. Varying concentrations of CelQuat® H-100 (a cellulosic derivative available from National Starch & Chemical, Inc.) were stripped onto the membrane to form three calibration lines having different concentrations. In particular, the concentrations utilized were 2.5 parts CelQuat® H-100 per million of the solution (ppm) (calibration #1), 5 ppm (calibration #2), and 20 ppm (calibration #3).

Pre-albumin (1 mg/ml, obtained from Biogenesis, Inc.) was stripped onto the membrane to form a detection line. The membrane was dried for 1 hour at a temperature of 37°C. A cellulosic fiber wicking pad (Millipore Co.) was attached to one end of the membrane. The laminated membrane was then cut into small half dipsticks.

The end of the membrane opposite to the wicking pad was applied to a test well that contained 30 microliters of 2% Tween 20, 10 microliters of red fluorescent microspheres conjugated with anti-prealbumin polyclonal antibody, and water. The mixture also contained varying concentrations of pre-albumin in phosphate buffer saline. In particular, the concentrations tested were 0 micrograms, 75 micrograms and 125 micrograms.

It was observed that the three calibration lines turned different intensities of red, where the calibration line #3 has the highest and line #1 has the lowest intensity. The intensity of the detection line in this competitive assay was inversely proportional to the test pre-albumin concentration. When there was no pre-albumin, the conjugate was captured by the detection line and the three calibration lines. With an increased amount of pre-albumin antigen, the detection line became less intense.

The line intensity was then read by a fluorescence reader and used to generate a calibration curve. The results are shown below in Table 1.

**Table 1: Calibration for Pre-albumin Detection with Line Intensity**

| | Signal Intensity | | |
|---|---|---|---|
| Calibration #1 | 1 | 1 | 1 |
| Calibration #2 | 10 | 10 | 10 |
| Calibration #3 | 20 | 20 | 20 |
| Detection Line | 20 | 10 | 0 |

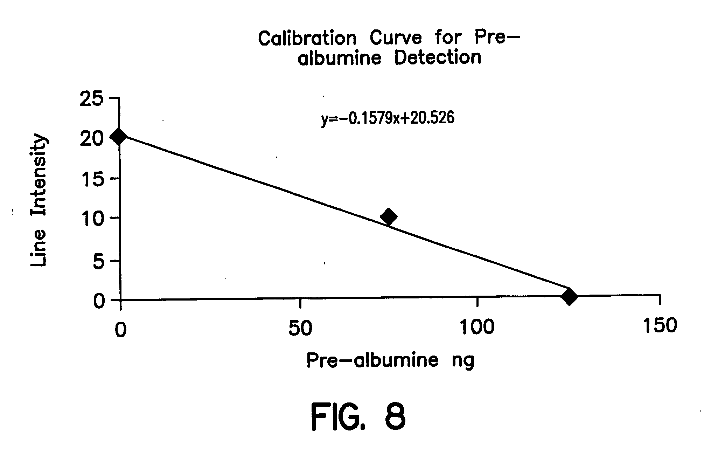

For the detection line, the signal intensity values of 20, 10, and 0 was determined to correspond to pre-albumin amounts of 0 micrograms, 75 micrograms, and 125 micrograms, respectively. A calibration curve generated from this data is also shown in Fig. 8. Using this calibration curve, the presence and/or amount of an unknown level of pre-albumin can be determined.

While the invention has been described in detail with respect to the specific embodiments thereof, it will be appreciated that those skilled in the art, upon attaining an understanding of the foregoing, may readily conceive of alterations to, variations of, and equivalents to these embodiments. Accordingly, the scope of the present invention should be assessed as that of the appended claims and any equivalents thereto.

## Claims

1. A flow-through assay device for detecting the presence or quantity of an analyte residing in a test sample said flow-through assay comprising a porous membrane, wherein said porous membrane is in fluid communication with a probe conjugate that contains a specific binding member and a detectable probe, said porous membrane defining:
a detection zone that contains an immobilized capture reagent that is capable of binding to the analyte or the probe conjugate, wherein said detection zone is capable of generating a detection signal that represents the presence or absence of the analyte,
a calibration zone that contains a polyelectrolyte binder configured to bind with said probe conjugate, said calibration zone including:
i) a first calibration region containing a first predetermined amount of said binder, said first calibration region being capable of generating a first calibration signal ;
ii) a second calibration region containing a second predetermined amount of said binder that is greater than said first predetermined amount of said binder said second calibration region being capable of generating a second calibration signal, said second calibration signal having a greater intensity than said first calibration signal ; and
wherein the relative amount of the analyte within the test sample is determined by comparing said detection signal to said first calibration signal and said second calibration signal.

2. A flow-through assay device as defined in claim 1, wherein said detection signal is capable of being visually compared to said first calibration signal and said second calibration signal.

3. A flow-through assay device as defined in claim 1, wherein said detection signal is capable of being compared to said first calibration signal and said second calibration signal through the use of an instrument.

4. A flow-through assay device as defined in claim 1, wherein a calibration curve is generated by plotting the intensity of said first and second calibration signals versus known levels of the analyte.

5. A flow-through assay device as defined in claim 1, wherein said calibration zone further includes a third calibration region containing a third predetermined amount of said binder that is greater than said second predetermined amount of said binder said third calibration region being capable of generating a third calibration signal that has a greater intensity than said second calibration signal.

6. A flow-through assay device as defined in claim 1, wherein said first and said second calibration regions are disposed in a direction that is parallel to the flow of the test sample through said porous membrane.

7. A flow-through assay device defined in claim 1, wherein said detectable probe is selected from the group consisting of chromogens, catalysts, fluorescent compounds,chemiluminescent compounds, radioactive labels, direct visual labels, liposomes, and combinations thereof.

8. A flow-through assay device as defined in claim 7, wherein said detectable probe comprises a latex microparticle.

9. A flow-through assay device as defined in claim 1, wherein the specific binding member of said probe conjugate is selected from the group consisting of antigens, haptens, antibodies, and complexes thereof.

10. A flow-through assay device as defined in claim 1, wherein the capture reagent is selected from the group consisting of antigens, haptens, antibodies, and complexes thereof.

11. A flow-through assay device as defined in claim 1, wherein the assay is a sandwich-type assay.

12. A flow-through assay device as defined in claim 1, wherein the assay is a competitive-type assay.

13. A flow-through assay device as defined in claim 1, wherein the capture reagent is capable of binding to the analyte, wherein the first and second calibration regions are first and second calibration lines; and wherein the calibration zone further includes iii) a third calibration line containing a third predetermined amount of said binder that is greater than said second predetermined amount of said binder, said third calibration line being capable of generating a third calibration signal that has a greater intensity than said second calibration signal.

14. A flow-through assay device as defined in claim 13, wherein said detection signal is capable of being visually compared to said first calibration signal, said second calibration signal, and said third calibration signal.

15. A flow-through assay device as defined in claim 13, wherein said detection signal is capable of being compared to said first calibration signal, said second calibration signal, and said third calibration signal through the use of an instrument.

16. A flow-through assay device as defined in claim 13, wherein a calibration curve is generated by plotting the intensity of said first, second, and third calibration signals versus known amounts of the analyte.

17. A flow-through assay device as defined in claim 13, wherein said first, second, and third calibration lines are disposed in a direction that is parallel to the flow of the test sample through said porous membrane

18. A flow-through assay device as defined in claim 1, wherein said probe conjugates being configured to combine with the analyte in the test sample when contacted therewith such that probe conjugate/analyte complexes and uncomplexed probe conjugates are formed, wherein the capture reagent is non-diffusively immobilized on said porous membrane, said capture reagent being capable of binding to said probe conjugate/analyte complexes, wherein the probe conjugates with which the binder is configured to bind are said uncomplexed probe conjugates.

19. A flow-through assay device as defined in claim 1, wherein the detection zone contains a predetermined amount of the capture reagent, and wherein the capture reagent is non-diffusively immobilized on said porous membrane, said capture reagent being capable of binding to said probe conjugates and to the analyte, and wherein said probe conjugates with which the binder binds are unbound to said capture reagents, wherein said specific binding member is identical to the analyte

20. A flow-through assay device as defined in claim 1, wherein said probe conjugates being configured to combine with the analyte in the test sample when contacted therewith such that probe conjugate/analyte complexes and uncomplexed probe conjugates are formed, wherein the capture reagent is non-diffusively immobilized on said porous membrane, said capture reagent being capable of binding to said uncomplexed probe conjugates, wherein the binder is configured to bind to said probe conjugate/analyte complexes and to said probe conjugates remaining unbound to said capture reagents, wherein said capture reagent is identical to the analyte.

21. A flow-through assay device as defined in claim 1, wherein the polyelectrolyte binder has a net positive charge.

22. A flow-through assay device as defined in claim 21, wherein the polyelectrolyte binder is selected from the group consisting of polylysine, polyethylenimine, epichlorohydrin-functionalized polyamines or polyamidoamines, polydiallyldimethyl-ammonium chloride, and cationic cellulose derivatives.

23. A flow-through assay device as defined in claim 1, wherein the polyelectrolyte binder has a net negative charge.

24. A flow-through assay device as defined in claim 1, wherein the polyelectrolyte binder is amphiphilic.

25. A flow-through assay device as defined in claim 1, wherein the polyelectrolyte binder is ionically bonded to a functional group present on the surface of the porous membrane.

26. A flow-through assay device as defined in claim 1, wherein the polyelectrolyte binder is covalently bonded to a functional group present on the surface of the porous membrane.

27. A flow-through assay device as defined in claim 26, wherein the polyelectrolyte binder is crosslinked to the functional group.

## Patentansprüche

1. Ein Durchflusstestgerät zum Detektieren des Vorhandenseins oder der Menge eines in einer Testprobe befindlichen Analyts, wobei das Durchflusstestgerät eine poröse Membran umfasst, wobei die poröse Membran in Fluidverbindung mit einem Sondenkonjugat ist, das ein spezifisches Bindeelement und eine detektierbare Sonde enthält, wobei die poröse Membran definiert:
eine Detektionszone, die einen immobilisierten Fangreagens enthält, der fähig ist, sich an den Analyt oder das Sondenkonjugat zu binden, wobei die Detektionszone fähig ist,
ein Detektionssignal zu erzeugen, das das Vorhandensein oder das Fehlen des Analyts darstellt;
eine Kalibrierungszone, die ein Polyelektrolytbindemittel enthält, das eingerichtet ist,
sich mit dem Sondenkonjugat zu binden, wobei die Kalibrierungszone umfasst:
i) einen ersten Kalibrierungsbereich, der eine erste vorbestimmte Menge des Bindemittels enthält, wobei der erste Kalibrierungsbereich fähig ist, ein erstes Kalibrierungssignal zu erzeugen;
ii) einen zweiten Kalibrierungsbereich, der eine zweite vorbestimmte Menge des Bindemittels enthält, welche größer als die erste vorbestimmte Menge des Bindemittels ist, wobei der zweite Kalibrierungsbereich fähig ist, ein zweites Kalibrierungssignal zu erzeugen, wobei das zweite Kalibrierungssignal eine größere Intensität aufweist als das erste Kalibrierungssignal; und
wobei die relative Menge des Analyts innerhalb der Testprobe bestimmt wird durch Vergleichen des Detektionssignals mit dem ersten Kalibrierungssignal und dem zweiten Kalibrierungssignal.

2. Ein Durchflusstestgerät wie im Anspruch 1 definiert, wobei das Detektionssignal fähig ist, visuell mit dem ersten Kalibrierungssignal und dem zweiten Kalibrierungssignal verglichen zu werden.

3. Ein Durchflusstestgerät wie im Anspruch 1 definiert, wobei das Detektionssignal fähig ist, mit dem ersten Kalibrierungssignal und dem zweiten Kalibrierungssignal durch Benutzung eines Gerätes verglichen zu werden.

4. Ein Durchflusstestgerät wie im Anspruch 1 definiert, wobei eine Kalibrierungskurve erzeugt wird durch Auftragen der Intensität des ersten und des zweiten Kalibrierungssignals gegen bekannte Grade des Analyts.

5. Ein Durchflusstestgerät wie im Anspruch 1 definiert, wobei die Kalibrierungszone weiter einen dritten Kalibrierungsbereich umfasst, der eine dritte vorbestimmte Menge des Bindemittels enthält, die größer als die zweite vorbestimmte Menge des Bindemittels ist, wobei der dritte Kalibrierungsbereich fähig ist, ein drittes Kalibrierungssignal zu erzeugen, das eine größere Intensität als das zweite Kalibrierungssignal aufweist.

6. Ein Durchflusstestgerät wie im Anspruch 1 definiert, wobei der erste und der zweite Kalibrierungsbereich in einer Richtung angeordnet sind, die parallel zum Fluss der Testprobe durch die poröse Membran ist.

7. Ein Durchflusstestgerät wie im Anspruch 1 definiert, wobei die detektierbare Sonde ausgewählt ist aus der Gruppe bestehend aus: Chromogenen, Katalysatoren, fluoreszierende Verbindungen, chemilumineszente Verbindungen, radioaktive Markierungen, direkt visuelle Markierungen, Liposomen und Kombinationen davon.

8. Ein Durchflusstestgerät wie im Anspruch 7 definiert, wobei die detektierbare Sonde ein Latexmikropartikel umfasst.

9. Ein Durchflusstestgerät wie im Anspruch 1 definiert, wobei das spezifische Bindeelement des Sondenkonjugats ausgewählt ist aus der Gruppe bestehend aus: Antigenen, Haptenen, Antikörpern und Komplexen davon.

10. Ein Durchflusstestgerät wie im Anspruch 1 definiert, wobei das Fangreagens ausgewählt ist aus der Gruppe bestehend aus: Antigenen, Haptenen, Antikörpern und Komplexen davon.

11. Ein Durchflusstestgerät wie im Anspruch 1 definiert, wobei das Testgerät ein Testgerät vom Sandwichtyp ist.

12. Ein Durchflusstestgerät wie im Anspruch 1 definiert, wobei das Testgerät ein Testgerät vom Kompetitivtyp ist.

13. Ein Durchflusstestgerät wie im Anspruch 1 definiert, wobei das Fangreagens fähig ist, sich an den Analyt zu binden, wobei der erste und der zweite Kalibrierungsbereich eine erste und zweite Kalibrierungslinie sind; und wobei die Kalibrierungszone weiter umfasst: (iii) eine dritte Kalibrierungslinie, die eine dritte vorbestimmte Menge des Bindemittels enthält, die größer als die zweite vorbestimmte Menge des Bindemittels ist, wobei die dritte Kalibrierungslinie fähig ist, eine drittes Kalibrierungssignal zu erzeugen, das eine größere Intensität als das zweite Kalibrierungssignal aufweist.

14. Ein Durchflusstestgerät wie im Anspruch 13 definiert, wobei das Detektionssignal fähig ist, visuell mit dem ersten Kalibrierungssignal, dem zweiten Kalibrierungssignal und dem dritten Kalibrierungssignal verglichen zu werden.

15. Ein Durchflusstestgerät wie im Anspruch 13 definiert, wobei das Detektionssignal fähig ist, mit dem ersten Kalibrierungssignal, dem zweiten Kalibrierungssignal und dem dritten Kalibrierungssignal durch Benutzung eines Geräts verglichen zu werden.

16. Ein Durchflusstestgerät wie im Anspruch 13 definiert, wobei eine Kalibrierungskurve erzeugt wird durch Auftragen der Intensität des ersten, zweiten und dritten Kalibrierungssignals gegen bekannte Mengen des Analyts.

17. Ein Durchflusstestgerät wie im Anspruch 13 definiert, wobei die erste, zweite und dritte Kalibrierungslinie in einer Richtung angeordnet sind, die parallel zum Fluss der Testprobe durch die poröse Membran ist.

18. Ein Durchflusstestgerät wie im Anspruch 1 definiert, wobei die Sondenkonjugate eingerichtet sind, sich mit dem Analyt in der Testprobe zu verbinden, wenn sie damit kontaktiert werden, so dass Sondenkonjugat/Analyt-Komplexe und nichtkomplexierte Sondenkonjugate gebildet werden, wobei das Fangreagens nichtdiffusiv auf der porösen Membran immobilisiert ist, wobei das Fangreagens fähig ist, sich an die Sondenkonjugat/Analyt-Komplexe zu binden, wobei die Sondenkonjugate, an die sich zu binden das Bindemittel eingerichtet ist, die nichtkomplexierten Sondenkonjugate sind.

19. Ein Durchflusstestgerät wie im Anspruch 1 definiert, wobei die Detektionszone eine vorbestimmte Menge des Fangreagens' enthält und wobei das Fangreagens nichtdiffusiv auf der porösen Membran immobilisiert ist, wobei das Fangreagens fähig ist, sich an die Sondenkonjugate und den Analyt zu binden, und wobei die Sondenkonjugate, an die sich das Bindemittel bindet, an die Fangreagenzien ungebunden sind, wobei das spezifische Bindeelement identisch mit dem Analyt ist.

20. Ein Durchflusstestgerät wie im Anspruch 1 definiert, wobei die Sondenkonjugate eingerichtet sind, sich mit dem Analyt in der Testprobe zu verbinden, wenn sie damit kontaktiert werden, so dass Sondenkonjugat/Analyt-Komplexe und nichtkomplexierte Sondenkonjugate gebildet werden, wobei das Fangreagens nichtdiffusiv auf der porösen Membran immobilisiert ist, wobei das Fangreagens fähig ist, sich an die nichtkomplexierten Sondenkonjugate zu binden, wobei das Bindemittel eingerichtet ist, sich an die Sondenkonjugat/Analyt-Komplexe zu binden und an die Sondenkonjugate, die mit den Fangreagenzien ungebunden verbleiben, wobei das Fangreagens identisch mit dem Analyt ist.

21. Ein Durchflusstestgerät wie im Anspruch 1 definiert, wobei das Polyelektrolytbindemittel eine Nettopositivladung aufweist.

22. Ein Durchflusstestgerät wie im Anspruch 21 definiert, wobei das Polyelektrolytbindemittel ausgewählt ist aus der Gruppe bestehend aus: Polylysin, Polyethylenimin, epichlorohydrin-funktionalisierten Polyaminen oder Polyamidoaminen, Polydiallyldimethyl-Ammoniumchlorid und kationischen Cellulosederivaten.

23. Ein Durchflusstestgerät wie im Anspruch 1 definiert, wobei das Polyelektrolytbindemittel eine Nettonegativladung aufweist.

24. Ein Durchflusstestgerät wie im Anspruch 1 definiert, wobei das Polyelektrolytbindemittel amphiphil ist.

25. Ein Durchflusstestgerät wie im Anspruch 1 definiert, wobei das Polyelektrolytbindemittel ionisch an eine funktionale Gruppe gebunden ist, die auf der Oberfläche der porösen Membran vorhanden ist.

26. Ein Durchflusstestgerät wie im Anspruch 1 definiert, wobei das Polyelektrolytbindemittel kovalent an eine funktionale Gruppe gebunden ist, die auf der Oberfläche der porösen Membran vorhanden ist.

27. Ein Durchflusstestgerät wie im Anspruch 26 definiert, wobei das Polyelektrolytbindemittel mit der funktionellen Gruppe quervernetzt ist.

## Revendications

1. Dispositif de dosage à renouvellement continu pour détecter la présence ou la quantité d'un analyte se trouvant dans un échantillon à tester, ledit dispositif de dosage à renouvellement continu comprenant une membrane poreuse, ladite membrane poreuse étant en communication de fluide avec un conjugué de sonde qui contient un élément de liaison spécifique et une sonde détectable, ladite membrane poreuse définissant :
une zone de détection qui contient un réactif de capture immobilisé capable de se lier à l'analyte ou au conjugué de sonde, ladite zone de détection étant capable de générer un signal de détection qui représente la présence ou l'absence de l'analyte ;
une zone d'étalonnage qui contient un liant polyélectrolyte configuré pour se lier avec ledit conjugué de sonde, ladite zone d'étalonnage incluant :
i) une première région d'étalonnage contenant une première quantité prédéterminée dudit liant, ladite première région d'étalonnage étant capable de générer un premier signal d'étalonnage ;
ii) une seconde région d'étalonnage contenant une seconde quantité prédéterminée dudit liant qui est supérieure à ladite première quantité prédéterminée dudit liant, ladite seconde région d'étalonnage étant capable de générer un second signal d'étalonnage, ledit second signal d'étalonnage ayant une intensité supérieure à celle dudit premier signal d'étalonnage ; et
la quantité relative de l'analyte au sein de l'échantillon à tester étant déterminée en comparant ledit signal de détection audit premier signal d'étalonnage et audit second signal d'étalonnage.

2. Dispositif de dosage à renouvellement continu selon la revendication 1, dans lequel ledit signal de détection peut être comparé visuellement audit premier signal d'étalonnage et audit second signal d'étalonnage.

3. Dispositif de dosage à renouvellement continu selon la revendication 1, dans lequel ledit signal de détection peut être comparé audit premier signal d'étalonnage et audit second signal d'étalonnage, à l'aide d'un instrument.

4. Dispositif de dosage à renouvellement continu selon la revendication 1, dans lequel une courbe d'étalonnage est générée en traçant l'intensité desdits premier et second signaux d'étalonnage par rapport à des quantités connues de l'analyte.

5. Dispositif de dosage à renouvellement continu selon la revendication 1, dans lequel ladite zone d'étalonnage inclut, en outre, une troisième région d'étalonnage contenant une troisième quantité prédéterminée dudit liant supérieure à ladite seconde quantité prédéterminée dudit liant, ladite troisième région d'étalonnage étant capable de générer un troisième signal d'étalonnage qui a une intensité supérieure à celle dudit second signal d'étalonnage.

6. Dispositif de dosage à renouvellement continu selon la revendication 1, dans lequel lesdites première et seconde régions d'étalonnage sont disposées selon une direction qui est parallèle à l'écoulement de l'échantillon à tester au travers de ladite membrane poreuse.

7. Dispositif de dosage à renouvellement continu selon la revendication 1, dans lequel ladite sonde détectable est sélectionnée dans le groupe consistant en les chromogènes, les catalyseurs, les composés fluorescents, les composés chimioluminescents, les marqueurs radioactifs, les marqueurs directement visibles, les liposomes, et leurs combinaisons.

8. Dispositif de dosage à renouvellement continu selon la revendication 7, dans lequel ladite sonde détectable comprend une microparticule de latex.

9. Dispositif de dosage à renouvellement continu selon la revendication 1, dans lequel l'élément de liaison spécifique dudit conjugué de sonde est sélectionné dans le groupe consistant en les antigènes, les haptènes, les anticorps et leurs complexes.

10. Dispositif de dosage à renouvellement continu selon la revendication 1, dans lequel le réactif de capture est sélectionné dans le groupe consistant en les antigènes, les haptènes, les anticorps et leurs complexes.

11. Dispositif de dosage à renouvellement continu selon la revendication 1, ledit dosage étant du type sandwich.

12. Dispositif de dosage à renouvellement continu selon la revendication 1, ledit dosage étant du type compétitif.

13. Dispositif de dosage à renouvellement continu selon la revendication 1, dans lequel le réactif de capture peut se lier à l'analyte, les première et seconde régions d'étalonnage sont des première et seconde lignes d'étalonnage et la zone d'étalonnage inclut en outre : iii) une troisième ligne d'étalonnage contenant une troisième quantité prédéterminée dudit liant qui est supérieure à la seconde quantité prédéterminée dudit liant, ladite troisième ligne d'étalonnage étant capable de générer un troisième signal d'étalonnage qui a une intensité supérieure à celle dudit second signal d'étalonnage.

14. Dispositif de dosage à renouvellement continu selon la revendication 13, dans lequel ledit signal de détection peut être comparé visuellement audit premier signal d'étalonnage, audit second signal d'étalonnage et audit troisième signal d'étalonnage.

15. Dispositif de dosage à renouvellement continu selon la revendication 13, dans lequel ledit signal de détection peut être comparé audit premier signal d'étalonnage, audit second signal d'étalonnage et audit troisième signal d'étalonnage, à l'aide d'un instrument.

16. Dispositif de dosage à renouvellement continu selon la revendication 13, dans lequel une courbe d'étalonnage est générée en traçant l'intensité desdits premier, second et troisième signaux d'étalonnage par rapport à des quantités connus de l'analyte.

17. Dispositif de dosage à renouvellement continu selon la revendication 13, dans lequel lesdites première, seconde et troisième lignes d'étalonnage sont disposées selon une direction qui est parallèle à l'écoulement de l'échantillon à tester au travers de ladite membrane poreuse.

18. Dispositif de dosage à renouvellement continu selon la revendication 1, dans lequel lesdits conjugués de sonde sont configurés pour se combiner avec l'analyte dans l'échantillon à tester lorsqu'ils viennent à son contact de telle sorte que sont formés des complexes conjugué de sonde/analyte et des conjugués de sonde non complexés, le réactif de capture étant immobilisé de façon non-diffusante sur ladite membrane poreuse, ledit réactif de capture pouvant se lier auxdits complexes conjugué de sonde/analyte, les conjugués de sonde avec lesquels se lie le liant étant lesdits conjugués de sonde non complexés.

19. Dispositif de dosage à renouvellement continu selon la revendication 1, dans lequel la zone de détection contient une quantité prédéterminée du réactif de capture et dans lequel le réactif de capture est immobilisé de façon non-diffusante sur ladite membrane poreuse, ledit réactif de capture pouvant se lier auxdits conjugués de sonde et à l'analyte, lesdits conjugués de sonde avec lesquels le liant se lie étant non-liés auxdits réactifs de capture, ledit élément de liaison spécifique étant identique à l'analyte.

20. Dispositif de dosage à renouvellement continu selon la revendication 1, dans lequel lesdits conjugués de sonde sont configurés pour se lier avec l'analyte contenu dans l'échantillon à tester lorsqu'ils viennent à son contact de telle sorte que sont formés des complexes conjugué de sonde/analyte et des conjugués de sonde non complexés, le réactif de capture étant immobilisé de façon non-diffusante sur ladite membrane poreuse, ledit réactif de capture pouvant se lier auxdits conjugués de sonde non complexés, le liant étant configuré pour se lier auxdits complexes conjugué de sonde/analyte et auxdits conjugués de sonde restant non liés auxdits réactifs de capture, ledit réactif de capture étant identique à l'analyte.

21. Dispositif de dosage à renouvellement continu selon la revendication 1, dans lequel le liant polyélectrolyte a une charge nette positive.

22. Dispositif de dosage à renouvellement continu selon la revendication 21, dans lequel le liant polyélectrolyte est sélectionné dans le groupe consistant en la polylysine, la polyéthylèneimine, les polyamines ou polyamidoamines fonctionnalisées par l'épichlorohydrine, le poly(diallyldiméthyl-chlorure d'ammonium) et les dérivés cationiques de la cellulose.

23. Dispositif de dosage à renouvellement continu selon la revendication 1, dans lequel le liant polyélectrolyte a une charge nette négative.

24. Dispositif de dosage à renouvellement continu selon la revendication 1, dans lequel le liant polyélectrolyte est amphiphile.

25. Dispositif de dosage à renouvellement continu selon la revendication 1, dans lequel le liant polyélectrolyte est lié ioniquement à un groupe fonctionnel présent à la surface de la membrane poreuse.

26. Dispositif de dosage à renouvellement continu selon la revendication 1, dans lequel le liant polyélectrolyte est lié de manière covalente à un groupe fonctionnel présent à la surface de la membrane poreuse.

27. Dispositif de dosage à renouvellement continu selon la revendication 26, dans lequel le liant polyélectrolyte est réticulé au groupe fonctionnel. '
